# EUROPEAN PATENT SPECIFICATION

(11) **EP 3 603 698 B1**
(45) Date of publication and mention of the grant of the patent: **27.03.2024**
(21) Application number: 18770763.3
(22) Date of filing: 31.01.2018
(51) Int. Cl.: A61M 5/142, G16H 40/63

(54) **PORTABLE MEDICAL APPLIANCE AND METHOD FOR CONTROLLING PORTABLE MEDICAL APPLIANCE**
TRAGBARES MEDIZINISCHES GERÄT UND VERFAHREN ZUR STEUERUNG EINES TRAGBAREN MEDIZINISCHEN GERÄTS
APPAREIL MÉDICAL PORTABLE ET PROCÉDÉ DE COMMANDE D'UN APPAREIL MÉDICAL PORTABLE

(30) Priority: 23.03.2017 JP 2017056942
(43) Date of publication of application: 05.02.2020
(73) Proprietor: Terumo Kabushiki Kaisha, Tokyo 151-0072 (JP)
(72) Inventor: TORAI, Yu, Ashigarakami-gun Kanagawa 259-0151 (JP)
(74) Representative: Casalonga
(86) International application number: PCT/JP2018/003217
(87) International publication number: WO 2018/173496

(56) References cited:
- WO-A1-2014/203533
- JP-A- 2011 508 637
- JP-A- 2012 521 805
- JP-A- 2015 173 915
- US-A1- 2011 149 759

## Description

### Technical Field

The present invention relates to a portable medical device and a control method of the portable medical device.

### Background Art

As a treatment for diabetic patients, a treatment of continuously infusing a small amount of insulin into the patient's body is performed. In this treatment, in order to continuously administer a drug solution (insulin) to a patient, there is used a portable drug solution administration device (so-called insulin pump) provided with a device body that is fixed to the patient's body or clothes to be used, and a remote controller to control the device body through wireless communication.

In order to suppress battery consumption on the device body side and reduce its battery capacity, such a portable medical device using wireless communication has a configuration in which wireless communication is started, with a transmission signal from the device body as a trigger, by the remote controller receiving the transmission signal, at the time of wireless communication. Furthermore, the wireless communication between the device body and the remote controller can be interrupted (see Patent Literature 1 below), by setting a communication setting between the device body and the remote controller to an in-flight mode when the user boards an airplane.

### Citation List

### Patent Literature

Patent Literature 1: US 2016/0,038,671 A

### Summary of Invention

### Technical Problem

However, in a case where no user interface is provided on the device body, the portable medical device configured as described above cannot restart the wireless communication without an operation of the remote controller, after the wireless communication between the device body and the remote controller is once interrupted. Therefore, although drug solution administration by the device body is continued, it becomes impossible to perform control such as setting change of administration and additional drug solution administration.

Accordingly, an object of the present invention is to provide a portable medical device and a control method of the portable medical device capable of restarting wireless communication even without an operation of a remote controller, even when wireless communication between a device body having no user interface and the remote controller to control the device body is stopped. Document US 2011/149759 A1 represents the closest prior art. Said document discloses equivalent features for establishing a communication link as the present invention, however does not disclose the features which allow interruption of communications for a selectable duration.

The invention is defined by the appended claims.

### Solution to Problem

The present invention for achieving such an object is a portable medical device including a device body and a remote controller that operates the device body. The device body includes: a power supply unit; a driving unit that performs a drug solution administration and is driven by electric power from the power supply unit; a main-body-side wireless communication unit that outputs identification information at a predetermined cycle when the power supply unit is turned on; and a main-body-side input/output control unit that controls the drug solution administration of the driving unit and the output of the main-body-side wireless communication unit, in accordance with information received by the main-body-side wireless communication unit. The remote controller includes: an input unit, wherein together with a stop request of output for the main-body-side wireless communication unit, a stop duration of the output or a restart time of the output; and a controller-side wireless communication unit that establishes wireless connection between with the main-body-side wireless communication unit that has outputted the identification information by receiving the identification information outputted from the main-body-side wireless communication unit, and outputs, to the main-body-side wireless communication unit, information that is related to stop of the output and inputted from the input unit. Advantageous Effects of Invention

According to the present invention having the configuration above, it becomes possible to restart wireless communication without an operation of the remote controller, even when wireless communication between the device body having no user interface and the remote controller to control the device body is stopped.

### Brief Description of Drawings

Fig. 1 is a schematic configuration view of a portable medical device of a first embodiment.
Fig. 2 is a block diagram for explaining the portable medical device of the first embodiment.
Fig. 3 is a flowchart (part 1) for explaining a control method of the portable medical device of the first embodiment.
Fig. 4 is a flowchart (part 2) for explaining the control method of the portable medical device of the first embodiment.
Fig. 5 is a time chart for explaining a state of wireless communication of the portable medical device of the first embodiment.
Fig. 6 is a schematic configuration view of a portable medical device of a second embodiment.
Fig. 7 is a block diagram for explaining the portable medical device of the second embodiment.
Fig. 8 is a flowchart for explaining a control method of the portable medical device of the second embodiment. Description of Embodiments

Hereinafter, each embodiment applied with the present invention will be described in detail with reference to the drawings. Note that, in each embodiment, a description is given to a configuration in a case where the present invention is applied to a drug solution administration device such as an insulin pump, as a portable medical device. However, the portable medical device of the present invention can be widely applied to portable medical devices configured by a device body and a remote controller to operate or monitor this device body through wireless communication, for example, such as a pain relief device or a biological information measurement device. Further, without limiting to the device body having no user interface as in the embodiment, even a device body having a user interface can restart wireless communication without performing an input operation.

### <<First Embodiment>>

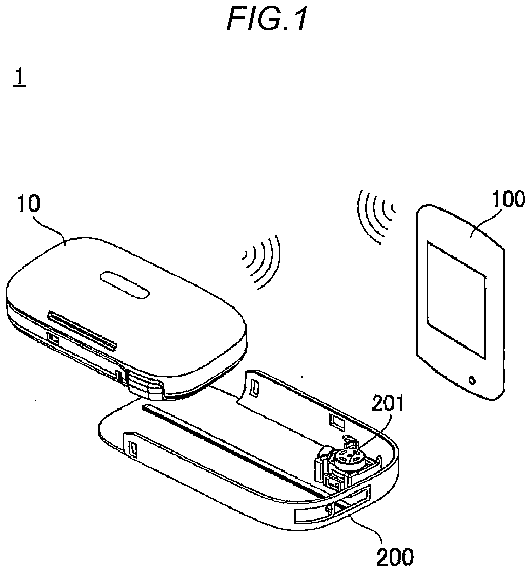

Fig. 1 is a schematic configuration view of a portable medical device of a first embodiment. A portable medical device 1 of the first embodiment shown in Fig. 1 is configured by a device body 10 and a remote controller 100 to operate the device body 10 through wireless communication.

Among them, the device body 10 is a pump unit to administrate a drug solution (insulin, as an example) to a patient for a long time, and is used by being worn on the skin of the patient via a cradle 200 that is to be bonded to the skin. Note that the cradle 200 includes a catheter port 201, and is used by being bonded to the skin of the patient. Whereas, the remote controller 100 is carried and used without being worn on the skin of the patient. Such a remote controller 100 is configured as a central device (master device) for operation of the device body 10 through wireless communication, when the device body 10 is a peripheral device (slave device).

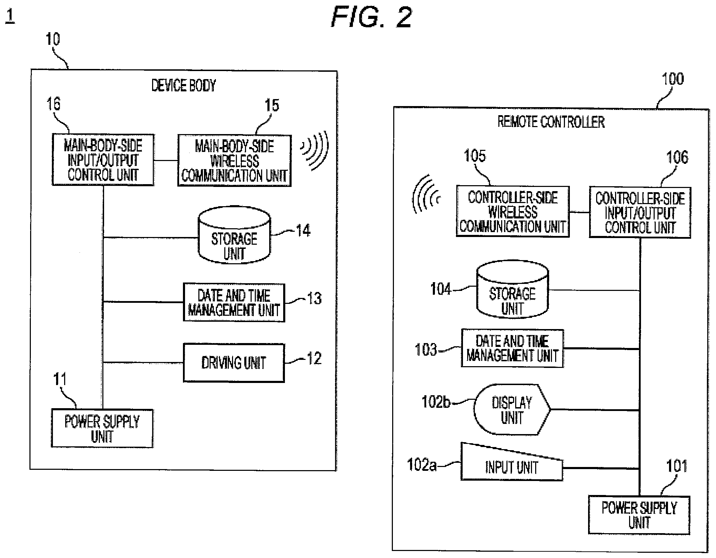

Fig. 2 is a block diagram for explaining the portable medical device of the first embodiment. Hereinafter, with reference to Fig. 2, details of a configuration of the portable medical device 1 will be described in the order of the device body 10 and the remote controller 100.

### <Device body 10>

In the device body 10, functions for performing administration of a drug solution (here, insulin as an example) are intensively arranged. Such a device body 10 includes a power supply unit 11, a driving unit 12, a date and time management unit 13, a storage unit 14, a main-body-side wireless communication unit 15, and a main-body-side input/output control unit 16 that controls these. However, the device body 10 is configured to have no user interface for operation of the driving unit 12 or the main-body-side wireless communication unit 15. Hereinafter, each element constituting the device body 10 is described.

### [Power supply unit 11 (Device body 10)]

The power supply unit 11 is for supplying electric power to each component constituting the device body 10, and is configured by, for example, a battery and a battery box to accommodate the battery. This device body 10 is configured such that power is turned on by, for example, mounting the battery to the battery box.

### [Driving unit 12 (Device body 10)]

The driving unit 12 is for performing a drug solution administration to a patient wearing the device body 10, and configured by a syringe to contain a drug solution to be administered, a pusher to be slid in the syringe, a motor and a gear mechanism for movement of the pusher, and the like. This driving unit 12 is configured to drive the motor by electric power from the power supply unit 11.

### [Date and time management Unit 13 (Device Body 10)]

The date and time management unit 13 is a program part to perform date and time management, which may be installed in a general microcomputer, and outputs date and time information.

### [Storage unit 14 (Device body 10)]

The storage unit 14 is a part to store various data. Data stored in the storage unit 14 is, for example, an administration condition of a drug solution transmitted from the remote controller 100 side, a wireless output stop setting, an administration history of a drug solution administration performed by the device body 10, and the like.

### [Main-body-side wireless communication unit 15 (Device body 10)]

The main-body-side wireless communication unit 15 is a part that performs wireless communication between with the remote controller 100. This main-body-side wireless communication unit 15 is a part that has a transmitter and a receiver and performs wireless communication using radio waves of a predetermined wavelength band, and in particular, performs wireless communication using ultra high frequency waves or microwaves. Here, in the main-body-side wireless communication unit 15, short-distance wireless communication with a communication distance of 100 m or less is to be performed. Specifically, a standardized wireless communication technology such as ZigBee (registered trademark IEEE 802.15.4: frequency 868 MHz band, 915 MHz band, 2.4 GHz band), Bluetooth (registered trademark: IEEE 802.15.1: frequency 2.4 GHz band), or wireless LAN (IEEE 802.11a/b/g/n/ac: frequency 2.4 GHz band, 5 GHz band) is applied.

In particular, the main-body-side wireless communication unit 15 is configured to periodically transmit identification information for causing the remote controller 100 to recognize the device body 10. Here, the device body 10 is configured to have no user interface for operation of the main-body-side wireless communication unit 15. Therefore, output of identification information by such a main-body-side wireless communication unit 15 is to be started with turning on of the power supply unit 11 as a trigger.

Further, such a main-body-side wireless communication unit 15 performs transmission and reception of signals between with the controller-side wireless communication unit 105 of the remote controller 100 that has received the identification information, and the main-body-side wireless communication unit 15 establishes connection for wireless communication between with the controller-side wireless communication unit 105.

Furthermore, in accordance with an instruction from the main-body-side input/output control unit 16, the main-body-side wireless communication unit 15 performs transmission and reception of an administration condition of a drug solution, a wireless output stop setting, an administration history of drug solution administration, and the like, between with the remote controller 100 with which the wireless communication connection has been established.

Such a main-body-side wireless communication unit 15 may be any one that performs wireless communication connection in the above-described procedure in particular, and for example, wireless communication using Bluetooth Low Energy (registered trademark) is performed.

Further, the device body 10 provided with such a main-body-side wireless communication unit 15 is configured as, with the remote controller 100 as a sensor device, a beacon device that transmits identification information of the device body 10 to the sensor device.

### [Main-body-side input/output control unit 16 (Device body 10)]

The main-body-side input/output control unit 16 is connected to the power supply unit 11, the driving unit 12, the date and time management unit 13, the storage unit 14, and the main-body-side wireless communication unit 15. This main-body-side input/output control unit 16 controls input and output of signals between these units, and controls drug solution administration by driving of the driving unit 12 and wireless communication by the main-body-side wireless communication unit 15, in accordance with information received by the main-body-side wireless communication unit 15.

Such a main-body-side input/output control unit 16 includes, for example, a central processing unit (CPU), a random access memory (RAM), and a read only memory (ROM), which are not shown here. The read only memory (ROM) may be the storage unit 14. An algorithm executed by such a main-body-side input/output control unit 16 for the control of drug solution administration from the driving unit 12 and wireless communication by the main-body-side wireless communication unit 15 will be described in detail in a control method of the portable medical device 1 below.

### <Remote controller 100>

The remote controller 100 operates the device body 10. In a case where this portable medical device 1 is an insulin pump, the remote controller 100 is used for setting basal dose of insulin by the device body 10, or optionally performing bolus dose of insulin by the device body 10 in accordance with an operation by the user.

Such a remote controller 100 includes a power supply unit 101, an input unit 102a, a display unit 102b, a date and time management unit 103, a storage unit 104, a controller-side wireless communication unit 105, and a controller-side input/output control unit 106 that controls these. Details of each component are as follows.

### [Power supply unit 101 (Remote controller 100)]

The power supply unit 101 is for supplying electric power to each component constituting the remote controller 100, and is configured by, for example, a battery and a battery box to accommodate the battery, as well as a switch that turns on and off the supply of electric power from the battery, and the like.

### [Input unit 102a (Remote controller 100)]

The input unit 102a is a user interface part to which the user inputs an on/off operation for power supply, an administration condition of a drug solution by the device body 10, a stop setting of wireless output from the main-body-side wireless communication unit 15, and selection of display contents on the display unit 102b. Such an input unit 102a may be, for example, a keypad or a touch panel provided on a front surface of the display unit 102b. In a case where the input unit 102a is a touch panel, the display unit 102b displays selection buttons, a ten key, and the like, for the touch panel.

Here, the wireless output stop setting for the main-body-side wireless communication unit 15 inputted from the input unit 102a is a stop request of wireless output, and a stop duration (for example, three hours) of the output from stopping to restarting of the wireless output or date and time (time of day) for restarting the wireless output. In addition, the wireless output stop setting inputted from the input unit 102a may be a time for stopping the wireless output, and a time period (for example, three hours) from stopping to restarting the wireless output or a time for restarting the wireless output. In this case, the time for stopping the wireless output is to be the stop request of the wireless output.

### [Display unit 102b (Remote controller 100)]

The display unit 102b displays various setting contents, input contents from the input unit 102a, a history of drug solution administration performed by the device body 10, and the like. Such a display unit 102b is configured with use of, for example, a liquid crystal display.

### [Date and time management unit 103 (Remote controller 100)]

The date and time management unit 103 may be similar to the date and time management unit 13 of the device body 10, and is a program part to perform date and time management.

### [Storage unit 104 (Remote controller 100)]

The storage unit 104 is a part to store various data. Data stored in the storage unit 104 is, for example, a history of drug solution administration transmitted from the device body 10 side, an administration condition of a drug solution inputted in the input unit 102a, a wireless output stop setting in the main-body-side wireless communication unit 15, and further, authentication data for communication connection with the device body 10, and the like. Note that the authentication data of communication connection may be identification information for recognizing the device body 10 in communication connection with this remote controller 100, and is also a link key created automatically by the communication connection.

### [Controller-side wireless communication unit 105 (Remote controller 100)]

The controller-side wireless communication unit 105 is a part that performs wireless communication between with the device body 10. This controller-side wireless communication unit 105 is configured by a transmission unit and a reception unit. This controller-side wireless communication unit 105 is applied with a standardized wireless communication technology similar to that of the main-body-side wireless communication unit 15.

Such a controller-side wireless communication unit 105 performs transmission and reception of signals between with the main-body-side wireless communication unit 15 of the device body 10 in communication connection, in accordance with an instruction from the controller-side input/output control unit 106, and the controller-side wireless communication unit 105 establishes connection of wireless communication between with the main-body-side wireless communication unit 15. Further, in accordance with an instruction from the controller-side input/output control unit 106, the controller-side wireless communication unit 105 transmits various settings inputted in the input unit 102a, to the main-body-side wireless communication unit 15 of the device body 10 in communication connection. Such a controller-side wireless communication unit 105 transmits a wireless output stop setting for the main-body-side wireless communication unit 15 inputted from the input unit 102a.

### [Controller-side input/output control unit 106 (Remote controller 100)]

The controller-side input/output control unit 106 is connected to the power supply unit 101, the input unit 102a, the display unit 102b, the date and time management unit 103, the storage unit 104, and the controller-side wireless communication unit 105. By controlling input and output of signals between these units, the controller-side input/output control unit 106 controls display on the display unit 102b and wireless communication by the controller-side wireless communication unit 105.

The controller-side input/output control unit 106 as described above includes, for example, a central processing unit (CPU), a random access memory (RAM), and a read only memory (ROM), which are not shown here. The read only memory (ROM) may be the storage unit 104. An algorithm for the control of wireless communication by the controller-side wireless communication unit 105 executed by such a controller-side input/output control unit 106 will be described in detail in the control method of the portable medical device 1 next.

### <Control method of portable medical device 1>

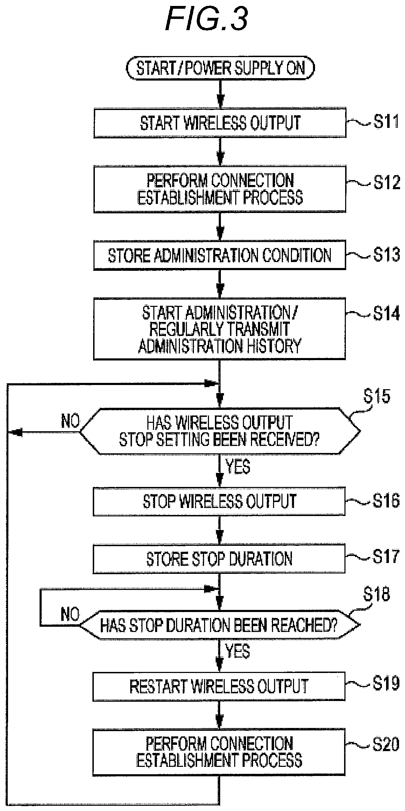

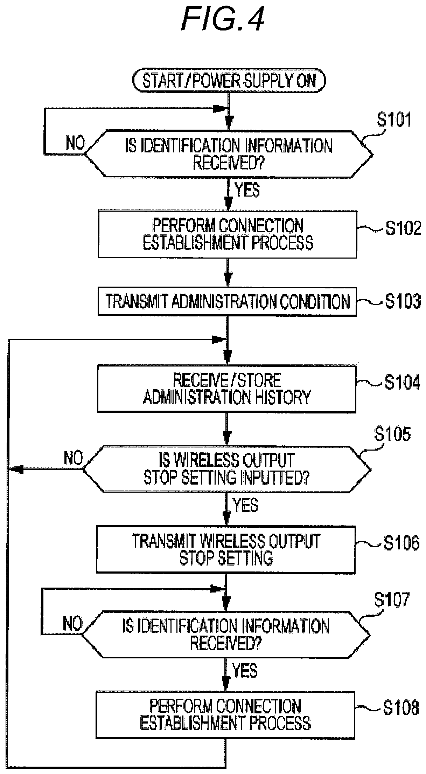

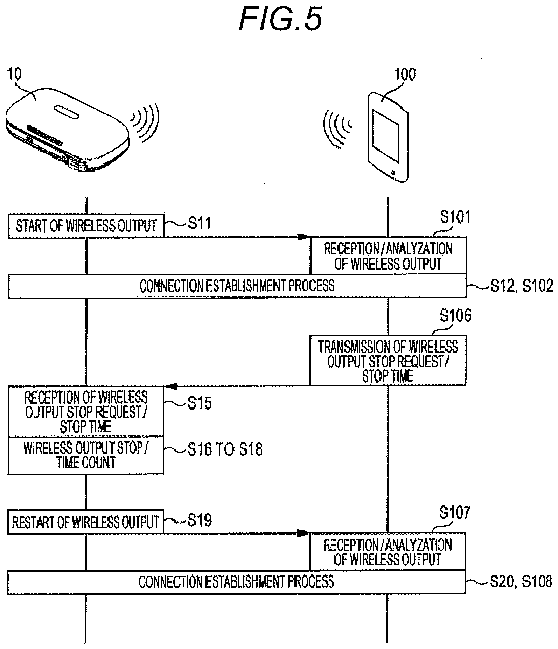

Fig. 3 is a flowchart (part 1) for explaining the control method of the portable medical device 1 of the first embodiment, and shows a procedure (steps S11 to S20) performed by the main-body-side input/output control unit 16 of the device body 10. Further, Fig. 4 is a flowchart (part 2) for explaining the control method of the portable medical device 1 of the first embodiment, and shows a procedure (steps S101 to S108) performed by the controller-side input/output control unit 106 of the remote controller 100. Furthermore, Fig. 5 is a time chart for explaining a state of wireless communication of the portable medical device 1 of the first embodiment.

The control method of the portable medical device 1 of the first embodiment described using these figures is a series of procedures in a case of assuming that wireless communication between the device body 10 and the remote controller 100 is temporarily stopped during drug solution administration by the device body 10. The procedure described below is realized by a CPU constituting the main-body-side input/output control unit 16 of the device body 10 and a CPU constituting the controller-side input/output control unit 106 of the remote controller 100 executing a program recorded in each ROM and RAM.

Hereinafter, the control method of the portable medical device 1 will be described in the order shown in the flowcharts of Figs. 3 and 4, with reference to Figs. 2 and 5.

### [Step S11 (Fig. 3)]

First, in step S11, when the power supply unit 11 is turned on by setting the battery in the battery box of the power supply unit 11, with this as a trigger, the main-body-side input/output control unit 16 starts wireless output from the main-body-side wireless communication unit 15. Here, the main-body-side wireless communication unit 15 continuously transmits, as wireless output, identification information for identifying the device body 10 at a constant cycle of, for example, 0.5 seconds.

### [Step S101 (Fig. 4)]

Whereas, in step S101, when the power supply unit 101 is turned on, for example, by an operation on the input unit 102a, with this as a trigger, the controller-side input/output control unit 106 starts signal reception by the controller-side wireless communication unit 105, and determines whether or not identification information from the main-body-side wireless communication unit 15 has been received. Here, the controller-side input/output control unit 106 analyzes the wireless output received by the controller-side wireless communication unit 105. Then, it is determined whether or not the received wireless output is identification information of the device body 10 in communication connection with this remote controller 100. Then, the determination is repeated until it is determined that this identification information has been received (YES), and the process proceeds to the next step S102 when it is determined that the identification information has been received (YES).

### [Step S102 (Fig. 4)]

Next, in step S102, the controller-side input/output control unit 106 performs a connection establishment process for establishing connection of wireless communication between the controller-side wireless communication unit 105 and the main-body-side wireless communication unit 15.

### [Step S12 (Fig. 3)]

Similarly, in step S12, the main-body-side input/output control unit 16 performs a connection establishment process for establishing connection of wireless communication between the controller-side wireless communication unit 105 and the main-body-side wireless communication unit 15.

### [Step S103 (Fig. 4)]

Thereafter, in step S103, the controller-side input/output control unit 106 causes the controller-side wireless communication unit 105 to transmit an administration condition of a drug solution by the driving unit 12 of the device body 10. This administration condition is information inputted by the user from the input unit 102a or information inputted by the user from the input unit 102a and stored in the storage unit 104.

### [Step S13 (Fig. 3)]

Whereas, in step S13, the main-body-side input/output control unit 16 stores the administration condition of the drug solution transmitted from the controller-side wireless communication unit 105 and received by the main-body-side wirelesscommunication unit 15, in the storage unit 14 as a set value.

### [Step S14 (Fig. 3)]

Thereafter, in step S14, the main-body-side input/output control unit 16 controls the driving unit 12 in accordance with the administration condition stored in the storage unit 14, to start drug solution administration. Further, when performing drug solution administration, the main-body-side input/output control unit 16 stores an administration history of the drug solution actually administered by the control of the driving unit 12, in the storage unit 14. Furthermore, the main-body-side input/output control unit 16 causes the main-body-side wireless communication unit 15 to regularly transmit the administration history of the drug solution stored in the storage unit 14, to the controller-side wireless communication unit 105.

### [Step S104 (Fig. 4)]

Whereas, in step S104, when the administration history transmitted from the main-body-side wireless communication unit 15 has been received by the controller-side wireless communication unit 105, the controller-side input/output control unit 106 stores the received administration history in the storage unit 104, and causes the display unit 102b to display as necessary.

The above procedure is a procedure of a normal drug solution administration by the portable medical device 1. Among these, step S14 performed by the device body 10 and step S104 performed by the remote controller 100 are continuously performed in accordance with the setting of the administration condition of the drug solution, stored in the storage unit 14 of the device body 10.

Then, a procedure described below is to be a procedure unique to the first embodiment. The procedure described below is a procedure in a case where it becomes necessary to stop wireless communication between the device body 10 and the remote controller 100 during execution of the normal drug solution administration, for example, such as a case where the user of the portable medical device 1 boards an aircraft in which in-flight wireless communication is prohibited, and the procedure is performed as follows.

### [Step S105 (Fig. 4)]

First, in step S105, the controller-side input/output control unit 106 determines whether or not a wireless output stop setting has been inputted from the input unit 102a. Then, when it is determined that there is an input (YES), the process proceeds to the next step S106. Whereas, when it is determined that there is no input (NO), the process returns to step S104.

Note that the determination as to whether or not a wireless output stop setting by the controller-side input/output control unit 106 has been inputted may be determination based on whether or not it is time for stopping the wireless output on the basis of setting information for stopping wireless output stored in advance in the storage unit 104.

### [Step S106 (Fig. 4)]

Next, in step S106, the controller-side input/output control unit 106 causes the controller-side wireless communication unit 105 to transmit a wireless output stop setting. The wireless output stop setting to be transmitted here is a stop request of wireless output and a length of a time period (for example, three hours) from stopping to restarting of the wireless output, or a time for starting the wireless output.

### [Step S15 (Fig. 3)]

Whereas, in step S15, the main-body-side input/output control unit 16 determines whether or not a wireless output stop setting has been received in the main-body-side wireless communication unit 15 and repeats the determination until it is determined as being received (YES), and the process proceeds to the next step S16 when it is determined as being received (YES).

### [Step S16 (Fig. 3)]

Next, in step S16, the main-body-side input/output control unit 16 stops the wireless output of the main-body-side wireless communication unit 15. This interrupts the wireless connection between the main-body-side wireless communication unit 15 and the controller-side wireless communication unit 105 established in step S12 and step S102. In this state, in order to restart the wireless connection between the main-body-side wireless communication unit 15 and the controller-side wireless communication unit 105, it is necessary to perform the connection establishment process again.

### [Step S17 (Fig. 3)]

Further, in step S17, the main-body-side input/output control unit 16 stores, among the wireless output stop setting received in step S15, the length of the time period (for example, three hours) from stopping to restarting of the wireless output or the time for restarting the wireless output, in the storage unit 14.

### [Step S18 (Fig. 3)]

Further, in step S18, the main-body-side input/output control unit 16 determines whether or not a stop duration or a restart time after stopping of the wireless output of the main-body-side wireless communication unit 15 has reached a set time period (or time of day) stored in the storage unit 14. Here, it is determined whether or not the time managed by the date and time management unit 13 corresponds to the length of the time period for stopping the wireless output stored in the storage unit 14 after stopping the wireless communication, or whether or not the time for restarting the wireless output has been reached.

Then, the determination is repeated until it is determined as being reached (YES), and the process proceeds to the next step S19 when it is determined as being reached (YES) .

### [Step S19 (Fig. 3)]

Next, in step S19, the main-body-side input/output control unit 16 restarts the wireless output from the main-body-side wireless communication unit 15. Here, the main-body-side wireless communication unit 15 continuously transmits, as wireless output, identification information for identifying the device body 10 at a constant cycle of, for example, 0.5 seconds.

### [Step S107 (Fig. 4)]

Whereas, in step S107, the controller-side input/output control unit 106 determines whether or not the controller-side wireless communication unit 105 has received identification information. Here, when the controller-side wireless communication unit 105 has received wireless output, the controller-side input/output control unit 106 analyzes the wireless output. Then, it is determined whether or not the received wireless output is identification information of the device body 10 in communication connection with this remote controller 100. Then, the determination is repeated until it is determined that this identification information has been received (YES), and the process proceeds to the next step S108 when it is determined that the identification information has been received (YES).

### [Step S108 (Fig. 4)]

Next, in step S108, the controller-side input/output control unit 106 performs a connection establishment process for establishing connection of wireless communication between the controller-side wireless communication unit 105 and the main-body-side wireless communication unit 15, and thereafter, the process returns to step S104.

### [Step S20 (Fig. 3)]

Similarly, in step S20, the main-body-side input/output control unit 16 performs a connection establishment process for establishing connection of wireless communication between the controller-side wireless communication unit 105 and the main-body-side wireless communication unit 15, and thereafter, the process returns to step S15.

### <Effect of first embodiment>

According to the first embodiment described above, for example, when the user boards an aircraft in which in-flight wireless communication is prohibited, wireless output can be automatically restarted after the lapse of a wireless stop duration or a restart time even when the wireless output of the device body 10 having no user interface is stopped by an operation of the remote controller 100. Therefore, it becomes possible again to change the setting of drug solution administration, and to perform additional drug solution administration, that is, bolus administration of insulin from the device body 10.

### <<Second Embodiment>>

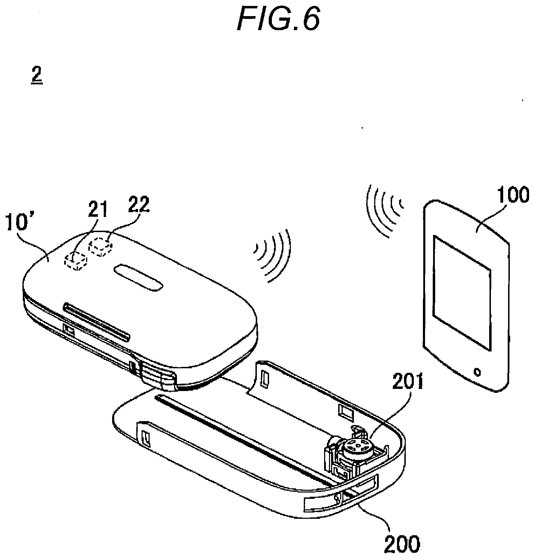

Fig. 6 is a schematic configuration view of a portable medical device 2 of a second embodiment. The portable medical device 2 of the second embodiment shown in Fig. 6 is different from the portable medical device 1 of the first embodiment described above in that a device body 10' includes an attachment detection unit 21 and a blockage detection unit 22 as a drive state detection unit. Further, a procedure of control performed by a main-body-side input/output control unit 16' and a controller-side input/output control unit 106' described later is different. Other configurations are similar to those in the first embodiment. Therefore, in the following, components similar to those in the first embodiment are denoted by the same reference numerals, and only configurations different from the first embodiment will be described while redundant description will be omitted.

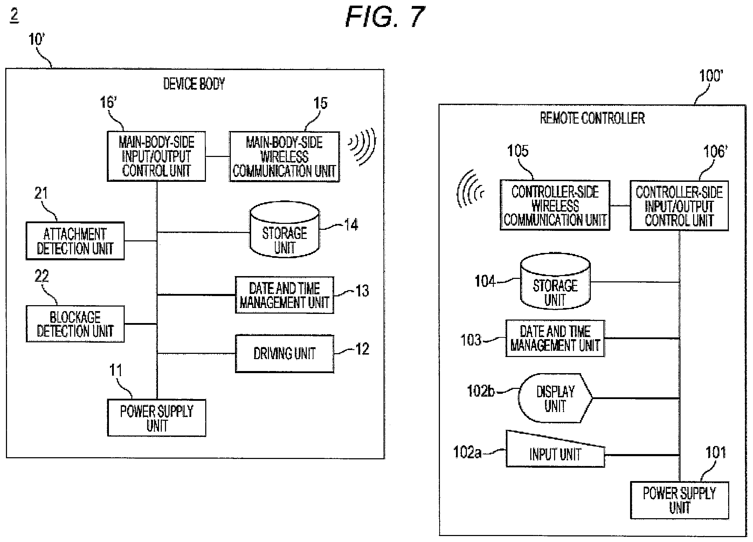

Fig. 7 is a block diagram for explaining the portable medical device of the second embodiment. Hereinafter, with reference to Fig. 7, features of a configuration of the device body 10' of the portable medical device 2 will be described.

### <Device body 10'>

In the device body 10', functions for performing administration of a drug solution (here, insulin as an example) are intensively arranged. Such a device body 10' includes a power supply unit 11, a driving unit 12, a date and time management unit 13, a storage unit 14, and a main-body-side wireless communication unit 15 similar to those of the first embodiment, and further includes the attachment detection unit 21 and the blockage detection unit 22. Further, the device body 10' includes the main-body-side input/output control unit 16' that controls these. Among them, the attachment detection unit 21, the blockage detection unit 22, and the main-body-side input/output control unit 16' having a configuration different from that of the first embodiment are as follows.

### [Attachment detection unit 21 (Device body 10')]

The attachment detection unit 21 is for detecting whether or not the device body 10' is attached to a cradle 200. As an example of such an attachment detection unit 21, for example, one is exemplified that is configured by a light emitting element and a light receiving element that detects reflected light of light emitted from the light emitting element. Such an attachment detection unit 21 has a configuration in which, in a state where the device body 10' is attached to the cradle 200, light emitted from the light emitting element is reflected by the cradle 200 and detected by the light receiving element, and whereby it is detected that the device body 10' is attached to the cradle 200.

Further, in particular, the attachment detection unit 21 also serves as input means in the device body 10'. That is, the attachment detection unit 21 switches on and off of a detection state of reflected light in the light receiving element by switching of attachment/non-attachment of the device body 10' to the cradle 200, that is, by attachment and removal of the device body 10' to and from the cradle 200. Therefore, for example, with a speed of this on/off switching, predetermined processing related to control of the main-body-side wireless communication unit 15 is associated. This association is stored in the storage unit 14.

For example, one switching operation of removing the device body 10' in a state of being attached to the cradle 200 to set in an non-attached state and attaching again within a predetermined time period (10 seconds) is associated with processing of extending a stop duration or a restart time of the wireless output of the main-body-side wireless communication unit 15, by a predetermined extension time (for example, 10 minutes).

Further, two switching operations within a predetermined time period (10 seconds) are associated with a trigger for forcibly restarting the wireless output of the main-body-side wireless communication unit 15.

Note that the attachment detection unit 21 is not limited to the one configured by the light emitting element and the light receiving element as described above, and may be, for example, one that detects an attachment state of the device body 10' to the cradle 200 by mechanical switching, a reed switch, or the like.

### [Blockage detection unit 22 (Device body 10')]

The blockage detection unit 22 is a drive state detection unit for detection of a drive failure of the driving unit 12. For example, in a case where the driving unit 12 is configured by a syringe to contain a drug solution to be administered, a pusher to be slid in the syringe, a motor and gear mechanism for movement of the pusher, and the like, this blockage detection unit 22 may be an encoder provided in the gear mechanism, as an example. In addition, the blockage detection unit 22 may detect a drive state by a pressure sensor or a flow rate sensor.

### [Main-body-side input/output control unit 16' (Device body 10')]

The main-body-side input/output control unit 16' is connected to the power supply unit 11, the driving unit 12, the date and time management unit 13, the storage unit 14, the main-body-side wireless communication unit 15, the attachment detection unit 21, and the blockage detection unit 22. This main-body-side input/output control unit 16' controls input and output of signals between these units, and controls drug solution administration by driving of the driving unit 12 and wireless communication by the main-body-side wireless communication unit 15, in accordance with information received by the main-body-side wireless communication unit 15 and signals from the attachment detection unit 21 and the blockage detection unit 22.

The fact that such a main-body-side input/output control unit 16' includes, for example, a central processing unit (CPU), a random access memory (RAM), and a read only memory (ROM), which are not shown here, is similar to the first embodiment.

Next, an algorithm executed by such a main-body-side input/output control unit 16' for the control of drug solution administration from the driving unit 12 and wireless communication by the main-body-side wireless communication unit 15 will be described as a control method of the portable medical device 2.

### <Control method of portable medical device 2>

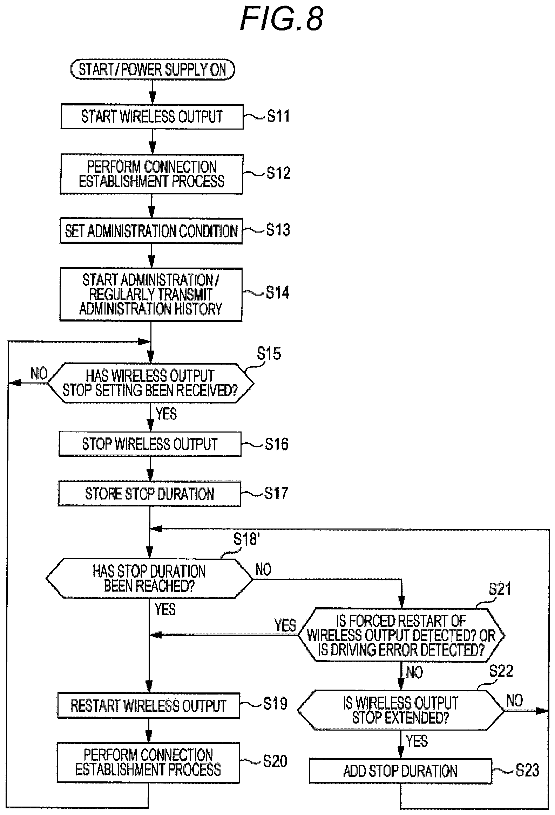

Fig. 8 is a flowchart for explaining the control method of the portable medical device 2 of the second embodiment, and shows a procedure to be performed by the main-body-side input/output control unit 16' of the device body 10'. A difference between the procedure shown in Fig. 8 and the procedure to be performed by the main-body-side input/output control unit 16 of the device body 10 of the first embodiment described using Fig. 3 is that steps S21 to S23 are added after the determination in step S18'. Other steps S11 to S20 are similar to the procedure described in the first embodiment. Further, a procedure to be performed by the controller-side input/output control unit 106 of the remote controller 100 is similar to steps S101 to S108 described using Fig. 4. Hereinafter, with reference to Fig. 7, a procedure different from that of the first embodiment will be described with reference to Fig. 8.

### [Step S18' (Fig. 8)]

In step S18', the main-body-side input/output control unit 16' determines whether or not a stop duration or a restart time after stopping of the wireless output of the main-body-side wireless communication unit 15 has reached a stop duration or a restart time stored in the storage unit 14. This determination is performed similarly to step S18 in the first embodiment, and the process proceeds to the next step S19 similarly to the first embodiment when it is determined as having reached (YES).

Whereas, when the main-body-side input/output control unit 16' determines that the stop duration or the restart time after stopping of the wireless output of the main-body-side wireless communication unit 15 has not reached the stop duration or the restart time stored in the storage unit 14 (NO), the process proceeds to step S21.

### [Step S21 (Fig. 8)]

In step S21, the main-body-side input/output control unit 16' makes two determinations as to whether or not forced restart of wireless output has been detected, and whether or not an error contrary to a setting condition has occurred in drug solution administration from the driving unit 12. The error includes blockage of a drug solution administration route, lack of a remaining drug solution, and the like.

That is, the main-body-side input/output control unit 16' determines whether or not there is detection associated with forced restart of the wireless output, in the attachment detection unit 21. Here, for example, it is determined whether or not an operation for removing the device body 10' in a state of being attached to the cradle 200 from the cradle 200 and attaching again has been detected twice within a predetermined time period (10 seconds) in the attachment detection unit 21.

Further, the main-body-side input/output control unit 16' determines whether or not an occurrence of blockage in an administration route of the drug solution from the driving unit 12 has been detected in the blockage detection unit 22.

When it is determined that either one is detected (YES), the process proceeds to step S19, the wireless output from the main-body-side wireless communication unit 15 is restarted, and identification information for identifying the device body 10' is continuously transmitted at a constant cycle of, for example, 0.5 seconds. Further, in the next step S20, a connection establishment process of wireless communication between the main-body-side wireless communication unit 15 and the controller-side wireless communication unit 105 is performed.

Then, although illustration is omitted here, when the blockage detection unit 22 has detected that blockage has occurred in an administration route of the drug solution from the driving unit 12, blockage occurrence information is transmitted from the main-body-side wireless communication unit 15 to the remote controller 100 in accordance with a normal procedure. With regard to notification of the blockage, a notification unit may be provided in the device body 10', and notification may be made by an alarm sound or vibration. This has the effect that the user can quickly recognize the abnormality. Also in this case, even without using the remote controller 100, it becomes possible to input a postponement or stop of communication restart of the device body 10' by an operation of the attachment detection unit 21.

Whereas, when the main-body-side input/output control unit 16' determines that none is detected in step S21 (NO), the process proceeds to step S22.

### [Step S22 (Fig. 8)]

In step S22, the main-body-side input/output control unit 16' determines whether or not extension of wireless output has been detected. Here, the main-body-side input/output control unit 16' determines whether or not there is detection associated with extension of wireless output, in the attachment detection unit 21. Here, for example, it is determined whether or not an operation for removing the device body 10' in a state of being attached to the cradle 200 from the cradle 200 and attaching again has been detected once within a predetermined time period (10 seconds) in the attachment detection unit 21.

When it is determined as being detected (YES), the process proceeds to step S23. Whereas, when it is determined as not being detected (NO), the process returns to step S18'.

### [Step S23 (Fig. 8)]

In step S23, the main-body-side input/output control unit 16' performs a process of adding a stop duration of the wireless output (or a time for restarting the wireless output) stored in the storage unit 14. Here, the stop duration of the wireless output (or the time for restarting the wireless output) stored in the storage unit 14 is rewritten to a time extended by a predetermined extension time (for example, 10 minutes). Thereafter, the process returns to step S18' to continue processing.

### <Effect of second embodiment>

According to the second embodiment described above, in addition to the effects of the first embodiment, in a state where the wireless output of the device body 10 having no user interface is stopped, it is possible forcibly restart the wireless output of the device body 10' and extend the stop of the wireless output, even without operation of the remote controller. Therefore, for example, when the user of the portable medical device 2 is on board an aircraft in which in-flight wireless communication is prohibited, the stop of the wireless output can be extended if arrival of the aircraft is delayed. In addition, even when an unexpected situation related to drug solution administration occurs while the wireless output is stopped, the wireless output of the device body 10 can be restarted urgently to enable the remote controller 100 to operate the device body 10.

It should be noted that the present invention is not limited to the embodiments described above and shown in the drawings, and various modifications can be made without departing from the subject matter of the invention described in the claims.

### Reference Signs List

- 1, 2: portable medical device
- 10, 10': device body
- 11: power supply unit
- 12: driving unit
- 15: main-body-side wireless communication unit
- 16: main-body-side input/output control unit
- 21: attachment detection unit
- 22: blockage detection unit (drive state detection unit)
- 100: remote controller
- 102a: input unit
- 105: controller-side wireless communication unit

## Claims

1. A portable medical device (1, 2) comprising a device body (10, 10') and a remote controller (100) that operates the device body (10, 10'), wherein
the device body (10, 10') comprises:
a power supply unit (11);
a driving unit (12) that performs a drug solution administration and is driven by electric power from the power supply unit (11);
a main-body-side wireless communication unit (15) that outputs identification information at a predetermined cycle when the power supply unit (11) is turned on; and
a main-body-side input/output control unit (16) that controls the drug solution administration of the driving unit (12) and the output of the main-body-side wireless communication unit (15), in accordance with information received by the main-body-side wireless communication unit (15), and
the remote controller (100) comprises:
an input unit (102a), wherein together with a stop request of the output for the main-body-side wireless communication unit (15), a stop duration of the output or a restart time of the output is inputted from the input unit (102a); and
a controller-side wireless communication unit (105) that establishes wireless connection between with the main-body-side wireless communication unit (15) that has outputted the identification information by receiving the identification information outputted from the main-body-side wireless communication unit (15), and outputs, to the main-body-side wireless communication unit (15), information that is related to stop of the output and inputted from the input unit (102a).

2. The portable medical device (1, 2) according to claim 1, wherein
the main-body-side input/output control unit (16) stops output of the main-body-side wireless communication unit (15), in accordance with a stop request of the output received by the main-body-side wireless communication unit (15), and restarts output of identification information from the main-body-side wireless communication unit (15) when a stop duration of the output or a restart time of the output has been reached, the stop duration or the restart time being received by the main-body-side wireless communication unit (15).

3. The portable medical device (1, 2) according to claim 1 or 2, wherein
the device body (10, 10') is used in a predetermined attachment state on a user, and comprises an attachment detection unit (21) that detects attachment/non-attachment on a user, and
by switching of attachment/non-attachment of the device body (10, 10') detected by the attachment detection unit (21), the main-body-side input/output control unit (16) causes the main-body-side wireless communication unit (15) being in a state where output is stopped to restart output of the identification information.

4. The portable medical device (1, 2) according to claim 1 or 2, wherein
the device body (10, 10') is used in a predetermined attachment state on a user, and comprises an attachment detection unit (21) that detects attachment/non-attachment on a user, and
the main-body-side input/output control unit (16) extends the stop duration or the restart time by switching of the attachment/non-attachment of the device body (10, 10') detected by the attachment detection unit (21).

5. The portable medical device (1, 2) according to claim 3 or 4, wherein
control of output of the main-body-side wireless communication unit (15) by the main-body-side input/output control unit (16) is performed to achieve a state associated with a speed of attachment/non-attachment switching of the device body (10, 10') detected by the attachment detection unit (21).

6. The portable medical device (1, 2) according to any one of claims 1 to 5, wherein
the device body (10, 10') comprises a drive state detection unit (22) that detects a drive failure of the driving unit (12), and
when the drive state detection unit (22) detects a drive failure of the driving unit (12), the main-body-side input/output control unit (16) causes the main-body-side wireless communication unit (15) being in a state where output is stopped to restart output of the identification information.

7. A control method of a portable medical device (1, 2) comprising:
a device body (10, 10') comprising a power supply unit (11), a driving unit (12) that performs a drug solution administration and is driven by electric power from the power supply unit (11), a main-body-side wireless communication unit (15), and a main-body-side input/output control unit (16) that controls the drug solution administration of the driving unit (12) and the output of the main-body-side wireless communication unit (15); and
a remote controller (100) that is to operate the device body (10, 10') and comprises an input unit (102a) and a controller-side wireless communication unit (105), the control method comprising:
outputting, by the main-body-side wireless communication unit (15), identification information at a predetermined cycle when the power supply unit (11) is turned on,
inputting, by the input unit (1 02a), together with a stop request of the output for the main-body-side wireless communication unit (15), a stop duration of the output or a restart time of the output,
establishing, by the controller-side wireless communication unit (105), wireless connection with the main-body-side wireless communication unit (15) that has outputted the identification information by receiving the identification information outputted from the main-body-side wireless communication unit (15), and outputting, to the main-body-side wireless communication unit (15), a stop request of the output inputted from the input unit (102a), and a stop duration of the output or a restart time of the output, and
stopping, by the main-body-side input/output control unit (16), output of the main-body-side wireless communication unit (15), in accordance with a stop request of the output received by the main-body-side wireless communication unit (15), and restarting output of identification information from the main-body-side wireless communication unit (15) when a stop duration of the output or a restart time of the output has been reached, the stop duration or the restart time being received by the main-body-side wireless communication unit (15).

## Patentansprüche

1. Tragbare medizinische Vorrichtung (1, 2), die einen Vorrichtungskörper (10, 10') und eine Fernsteuerung (100) umfasst, welche den Vorrichtungskörper (10, 10') betätigt, wobei
der Vorrichtungskörper (10, 10') umfasst:
eine Energieversorgungseinheit (11);
eine Antriebseinheit (12), die eine Verabreichung einer Arzneimittellösung durchführt und durch elektrische Energie von der Energieversorgungseinheit (11) angetrieben wird;
eine auf der Hauptkörperseite liegende drahtlose Kommunikationseinheit (15), die Identifikationsinformationen in einem vorbestimmten Zyklus ausgibt, wenn die Energieversorgungseinheit (11) eingeschaltet ist; und
eine auf der Hauptkörperseite liegende Eingabe/Ausgabe-Steuerungseinheit (16), welche die Verabreichung einer Arzneimittellösung von der Antriebseinheit (12) und die Ausgabe der auf der Hauptkörperseite liegenden drahtlosen Kommunikationseinheit (15) in Übereinstimmung mit von der auf der Hauptkörperseite liegenden drahtlosen Kommunikationseinheit (15) empfangenen Informationen steuert, und
die Fernsteuerung (100) umfasst:
eine Eingabeeinheit (102a), wobei zusammen mit einer Stoppanforderung der Ausgabe für die auf der Hauptkörperseite liegende drahtlose Kommunikationseinheit (15) eine Stoppdauer der Ausgabe oder eine Neustartzeit der Ausgabe von der Eingabeeinheit (102a) eingegeben wird; und
eine auf der Steuerungsseite liegende drahtlose Kommunikationseinheit (105), die eine drahtlose Verbindung mit der auf der Hauptkörperseite liegenden drahtlosen Kommunikationseinheit (15) herstellt, welche die Identifizierungsinformationen ausgegeben hat, indem sie die von der auf der Hauptkörperseite liegenden drahtlosen Kommunikationseinheit (15) ausgegebenen Identifizierungsinformationen empfängt, und an die auf der Hauptkörperseite liegende drahtlose Kommunikationseinheit (15) Informationen ausgibt, die sich auf den Stopp der Ausgabe beziehen und die von der Eingabeeinheit (102a) eingegeben werden.

2. Tragbare medizinische Vorrichtung (1, 2) nach Anspruch 1, wobei
die auf der Hauptkörperseite liegende Eingabe/Ausgabe-Steuerungseinheit (16) die Ausgabe der auf der Hauptkörperseite liegenden drahtlosen Kommunikationseinheit (15) in Übereinstimmung mit einer Stoppanforderung der Ausgabe, die von der auf der Hauptkörperseite liegenden drahtlosen Kommunikationseinheit (15) empfangen wird, stoppt und die Ausgabe von Identifikationsinformationen von der auf der Hauptkörperseite liegenden drahtlosen Kommunikationseinheit (15) wieder zu starten, wenn eine Stoppdauer der Ausgabe oder eine Neustartzeit der Ausgabe erreicht worden ist, wobei die Stoppdauer oder die Neustartzeit von der auf der Hauptkörperseite liegenden drahtlosen Kommunikationseinheit (15) empfangen wird.

3. Tragbare medizinische Vorrichtung (1, 2) nach Anspruch 1 oder 2, wobei
der Vorrichtungskörper (10, 10') in einem vorbestimmten Befestigungszustand an einem Benutzer verwendet wird und eine Befestigungserfassungseinheit (21) umfasst, welche die Befestigung/Nicht-Befestigung an einem Benutzer erfasst, und
durch Umschalten der Befestigung/Nicht-Befestigung des Vorrichtungskörpers (10, 10'), die von der Befestigungserkennungseinheit (21) erkannt wird, veranlasst die auf der Hauptkörperseite liegende Eingabe/Ausgabe-Steuerungseinheit (16) die auf der Hauptkörperseite liegende drahtlose Kommunikationseinheit (15), die sich in einem Zustand befindet, in dem die Ausgabe gestoppt ist, die Ausgabe der Identifikationsinformationen wieder zu starten.

4. Tragbare medizinische Vorrichtung (1, 2) nach Anspruch 1 oder 2, wobei
der Vorrichtungskörper (10, 10') in einem vorbestimmten Befestigungszustand an einem Benutzer verwendet wird und eine Befestigungserfassungseinheit (21) umfasst, welche die Befestigung/Nicht-Befestigung an einem Benutzer erfasst, und
die auf der Hauptkörperseite liegende Eingabe/Ausgabe-Steuerungseinheit (16) die Stoppdauer oder die Neustartzeit durch Umschalten der von der Befestigungserfassungseinheit (21) erfassten Befestigung/Nicht-Befestigung des Vorrichtungskörpers (10, 10') verlängert.

5. Tragbare medizinische Vorrichtung (1, 2) nach Anspruch 3 oder 4, wobei
die Steuerung der Ausgabe der auf der Hauptkörperseite liegenden drahtlosen Kommunikationseinheit (15) durch die auf der Hauptkörperseite liegende Eingabe-/Ausgabe-Steuerungseinheit (16) durchgeführt wird, um einen Zustand zu erreichen, der mit einer von der Befestigungserfassungseinheit (21) erfassten Geschwindigkeit der Umschaltung der Befestigung/Nicht-Befestigung des Vorrichtungskörpers (10, 10') verbunden ist.

6. Tragbare medizinische Vorrichtung (1, 2) nach einem der Ansprüche 1 bis 5, wobei
der Vorrichtungskörper (10, 10') eine Einheit (22) zur Erkennung des Antriebszustands umfasst, die einen Antriebsfehler der Antriebseinheit (12) erkennt, und
wenn die Einheit (22) zur Erkennung des Antriebszustands einen Antriebsfehler der Antriebseinheit (12) erkennt, veranlasst die auf der Hauptkörperseite liegende Eingabe-/Ausgabesteuerungseinheit (16) die auf der Hauptkörperseite liegende drahtlose Kommunikationseinheit (15), die sich in einem Zustand befindet, in dem die Ausgabe gestoppt ist, die Ausgabe der Identifikationsinformationen wieder neu zu starten.

7. Steuerungsverfahren für eine tragbare medizinische Vorrichtung (1, 2), umfassend:
einen Vorrichtungskörper (10, 10'), der eine Energieversorgungseinheit (11), eine Antriebseinheit (12), die eine Verabreichung einer Arzneimittellösung durchführt und durch elektrische Energie von der Energieversorgungseinheit (11) angetrieben wird, eine auf der Hauptkörperseite liegende drahtlose Kommunikationseinheit (15) und eine auf der Hauptkörperseite liegende Eingabe/Ausgabe-Steuerungseinheit (16), welche die Verabreichung der Arzneimittellösung durch die Antriebseinheit (12) und die Ausgabe der auf der Hauptkörperseite liegenden drahtlosen Kommunikationseinheit (15) steuert, umfasst; und
eine Fernsteuerung (100), die den Vorrichtungskörper (10, 10') betätigen soll und eine Eingabeeinheit (102a) und eine auf der Steuerungsseite liegende drahtlose Kommunikationseinheit (105) umfasst, wobei das Steuerungsverfahren umfasst;
das Ausgeben von Identifikationsinformationen durch die auf der Hauptkörperseite liegende drahtlose Kommunikationseinheit (15) in einem vorbestimmten Zyklus, wenn die Energieversorgungseinheit (11) eingeschaltet ist,
das Eingeben, durch die Eingabeeinheit (102a), zusammen mit einer Stoppanforderung der Ausgabe für die auf der Hauptkörperseite liegende drahtlose Kommunikationseinheit (15), einer Stoppdauer der Ausgabe oder einer Neustartzeit der Ausgabe,
das Herstellen einer drahtlosen Verbindung mit der auf der Hauptkörperseite liegenden drahtlosen Kommunikationseinheit (15), welche die Identifikationsinformationen ausgegeben hat, durch die auf der Steuerungsseite liegende drahtlose Kommunikationseinheit (105), indem die von der auf der Hauptkörperseite liegenden drahtlosen Kommunikationseinheit (15) ausgegebenen Identifikationsinformationen empfangen werden, und das Ausgeben einer Stoppanforderung von der Ausgabe, die durch die Eingabeeinheit (102a) eingegeben wurde, und einer Stoppdauer der Ausgabe oder einer Neustartzeit der Ausgabe an die auf der Hauptkörperseite liegende drahtlose Kommunikationseinheit (15), und
das Stoppen der Ausgabe der auf der Hauptkörperseite liegenden drahtlosen Kommunikationseinheit (15) durch die auf der Hauptkörperseite liegende Eingabe/Ausgabe-Steuerungseinheit (16), in Übereinstimmung mit einer Stoppanforderung der Ausgabe, die von der auf der Hauptkörperseite liegenden drahtlosen Kommunikationseinheit (15) empfangen wurde, und die Ausgabe von Identifikationsinformationen von der auf der Hauptkörperseite liegenden drahtlosen Kommunikationseinheit (15) wieder zu starten, wenn eine Stoppdauer der Ausgabe oder eine Neustartzeit der Ausgabe erreicht worden ist, wobei die Stoppdauer oder die Neustartzeit von der auf der Hauptkörperseite liegenden drahtlosen Kommunikationseinheit (15) empfangen wird.

## Revendications

1. Dispositif médical portable (1, 2) comprenant un corps de dispositif (10, 10') et un contrôleur à distance (100) qui fait fonctionner le corps de dispositif (10, 10'), dans lequel
le corps du dispositif (10, 10') comprend :
une unité d'alimentation électrique (11) ;
une unité d'entraînement (12) qui effectue une administration de solution médicamenteuse et qui est entraînée par l'énergie électrique provenant de l'unité d'alimentation (11) ;
une unité de communication sans fil côté corps principal (15) qui émet des informations d'identification à un cycle prédéterminé lorsque l'unité d'alimentation électrique (11) est allumée ; et
une unité de commande d'entrée/sortie côté corps principal (16) qui commande l'administration de la solution médicamenteuse de l'unité d'entraînement (12) et la sortie de l'unité de communication sans fil côté corps principal (15), en fonction des informations reçues par l'unité de communication sans fil côté corps principal (15), et
le contrôleur à distance (100) comprend :
une unité d'entrée (102a), dans lequel, en même temps qu'une demande d'arrêt de la sortie pour l'unité de communication sans fil côté corps principal (15), une durée d'arrêt de la sortie ou une heure de redémarrage de la sortie est entré à partir de l'unité d'entrée (102a) ; et
une unité de communication sans fil côté contrôleur (105) qui établit une connexion sans fil avec l'unité de communication sans fil côté corps principal (15) qui a émis les informations d'identification en recevant les informations d'identification émises par l'unité de communication sans fil côté corps principal (15), et émet, vers l'unité de communication sans fil côté corps principal (15), des informations liées à l'arrêt de l'émission et entrées par l'unité d'entrée (102a).

2. Dispositif médical portable (1, 2) selon la revendication 1, dans lequel l'unité de commande d'entrée/sortie côté corps principal (16) arrête la sortie de l'unité de communication sans fil côté corps principal (15), conformément à une demande d'arrêt de la sortie reçue par l'unité de communication sans fil côté corps principal (15), et redémarre la sortie des informations d'identification de l'unité de communication sans fil côté corps (15) lorsqu'une durée d'arrêt de la sortie ou une heure de redémarrage de la sortie a été atteint, la durée d'arrêt ou l'heure de redémarrage étant reçus par l'unité de communication sans fil côté corps (15).

3. Dispositif médical portable (1, 2) selon la revendication 1 ou la revendication 2, dans lequel le corps du dispositif (10, 10') est utilisé dans un état de fixation prédéterminé sur un utilisateur, et comprend une unité de détection de fixation (21) qui détecte la fixation/le non-attachement sur un utilisateur, et
par commutation de l'attachement/non-attachement du corps du dispositif (10, 10') détecté par l'unité de détection d'attachement (21), l'unité de commande d'entrée/sortie côté corps principal (16) fait en sorte que l'unité de communication sans fil côté corps principal (15) étant dans un état où la sortie est arrêtée, redémarre la sortie des informations d'identification.

4. Dispositif médical portable (1, 2) selon la revendication 1 ou la revendication 2, dans lequel le corps du dispositif (10, 10') est utilisé dans un état de fixation prédéterminé sur un utilisateur, et comprend une unité de détection de fixation (21) qui détecte la fixation/le non-attachement sur un utilisateur, et
l'unité de commande d'entrée/sortie côté corps principal (16) prolonge la durée d'arrêt ou l'heure de redémarrage par la commutation de l'attachement/non-attachement du corps du dispositif (10, 10') détecté par l'unité de détection de fixation (21).

5. Dispositif médical portable (1, 2) selon la revendication 3 ou la revendication 4, dans lequel la commande de la sortie de l'unité de communication sans fil côté corps principal (15) par l'unité de commande d'entrée/sortie côté corps principal (16) est effectuée pour atteindre un état associé à une vitesse de commutation de fixation/non fixation du corps du dispositif (10, 10') détectée par l'unité de détection de fixation (21).

6. Dispositif médical portable (1, 2) selon l'une quelconque des revendications 1 à 5, dans lequel le corps du dispositif (10, 10') comprend une unité de détection d'état d'entraînement (22) qui détecte une défaillance d'entraînement de l'unité d'entraînement (12), et
lorsque l'unité de détection de l'état de l'entraînement (22) détecte une défaillance de l'entraînement de l'unité d'entraînement (12), l'unité de commande d'entrée/sortie côté corps principal (16) fait en sorte que l'unité de communication sans fil côté corps principal (15) se trouve dans un état où la sortie est arrêtée pour redémarrer la sortie des informations d'identification.

7. Procédé de commande d'un dispositif médical portable (1, 2) comprenant :
un corps de dispositif (10, 10') comprenant une unité d'alimentation électrique (11), une unité d'entraînement (12) qui effectue une administration de solution médicamenteuse et est entraînée par l'énergie électrique provenant de l'unité d'alimentation électrique (11), une unité de communication sans fil côté corps principal (15), et une unité de commande d'entrée/sortie côté corps principal (16) qui commande l'administration de la solution médicamenteuse de l'unité d'entraînement (12) et la sortie de l'unité de communication sans fil côté corps principal (15) ; et un contrôleur à distance (100) qui doit faire fonctionner le corps du dispositif (10, 10') et qui comprend une unité d'entrée (102a) et une unité de communication sans fil côté contrôleur (105), le procédé de commande comprenant :
l'émission, par l'unité de communication sans fil côté corps principal (15), d'informations d'identification à un cycle prédéterminé lorsque l'unité d'alimentation électrique (11) est allumée,
la saisie, par l'unité d'entrée (102a), en même temps qu'une demande d'arrêt de la sortie pour l'unité de communication sans fil côté corps (15), d'une durée d'arrêt de la sortie ou d'une heure de redémarrage de la sortie,
l'établissement, au moyen de l'unité de communication sans fil côté contrôleur (105), d'une connexion sans fil avec l'unité de communication sans fil côté corps principal (15) qui a émis les informations d'identification en recevant les informations d'identification émises par l'unité de communication sans fil côté corps principal (15), et l'émission, vers l'unité de communication sans fil côté corps principal (15), d'une demande d'arrêt de la sortie émise par l'unité d'entrée (102a), et d'une durée d'arrêt de la sortie ou d'une heure de redémarrage de la sortie, et l'arrêt, au moyen de l'unité de commande d'entrée/sortie côté corps (16), de la sortie de l'unité de communication sans fil côté corps (15), conformément à une demande d'arrêt de la sortie reçue par l'unité de communication sans fil côté corps (15), et le redémarrage de la sortie des informations d'identification de l'unité de communication sans fil côté corps (15) lorsqu'une durée d'arrêt de la sortie ou une heure de redémarrage de la sortie a été atteinte, la durée d'arrêt ou l'heure de redémarrage étant reçues par l'unité de communication sans fil côté corps (15).
